# EUROPÄISCHE PATENTANMELDUNG

(11) **EP 1 623 690 A2**
(43) Veröffentlichungstag der Anmeldung: **08.02.2006**
(21) Anmeldenummer: 05013070.7
(22) Anmeldetag: 17.06.2005
(51) Int. Cl.: A61F 9/02, C25D 5/56, B29D 12/02

(54) **Laserschutzbrille bzw. Aufsetzrahmen für eine Laserschutzbrille**

(30) Priorität: 04.08.2004 DE 102004037671
(71) Anmelder: Uvex Arbeitsschutz GmbH, 90766 Fürth (DE)
(72) Erfinder: Herrmann, Herbert, 86736 Auhausen (DE); Hirschmann, Peter, Dr., 90768 Fürth (DE); Fröhlich, Thomas, Dr., 91460 Baudenbach (DE)
(74) Vertreter: Schneck, Herbert

(57) **Zusammenfassung**

Bei einer Laserschutzbrille bzw. einem Aufsetzrahmen für eine Laserschutzbrille umfassend einen Rahmen aus Kunststoff mit einer Metallbeschichtung, ist vorgesehen, dass die Metallbeschichtung durch Galvanisieren aufgebracht ist.

## Beschreibung

Die Erfindung betrifft eine Laserschutzbrille, umfassend einen Rahmen aus Kunststoff mit einer Metallbeschichtung.

Statt einer vollständigen Laserschutzbrille, welche eine oder zwei Scheiben umfasst, kann auch ein Aufsetzrahmen, insbesondere ein Aufsteckrahmen, vorgesehen sein, der für höhere Belastungen auf eine herkömmliche Laserschutzbrille aufgesetzt werden kann. Im Folgenden ist der Einfachheit halber stets nur von einer Laserschutzbrille die Rede, wenngleich auch ein Aufsetzrahmen mit umfasst sein soll.

Herkömmlicherweise werden die Rahmen von Laserschutzbrillen aus Kunststoff hergestellt und dann mit Aluminiumblechen bzw. Folien kaschiert. Hierzu müssen passende Aluminiumzuschnitte hergestellt und in arbeitsaufwändiger Weise aufkaschiert werden. Es ist auch bekannt, die Kunststoffe mit Füllmaterialien, wie z.B. Metallpulvern, zu versetzen, wobei aber keine für höhere Anforderungen ausreichende Beständigkeit erzielt wird.

Bei Skibrillen ist es aus optischen Gründen bekannt, eine metallische Lackierung vorzusehen. Schutzbrillen gegen Mikrowellen werden mit Metallfadengeweben ausgestattet.

Hiervon ausgehend liegt der Erfindung die Aufgabe zugrunde, eine Laserschutzbrille zu schaffen, die kostengünstig herstellbar ist und eine hohe Beständigkeit gegen Laserstrahlen aufweist.

Diese Aufgabe wird erfindungsgemäß dadurch gelöst, dass die Metallbeschichtung durch Galvanisieren erstellt ist.

Auf diese Weise entfällt die Notwendigkeit der Anfertigung passender Zuschnitte und das Aufkaschieren dieser Zuschnitte und es wird eine äußerst gleichmäßige, durchgehende Beschichtung realisiert, die eine sehr gute Schutzwirkung aufweist und auch optisch ansprechend ist.

Vorzugsweise wird ein Kunststoff verwendet, der sich gut zum Aufbringen einer galvanischen Beschichtung eignet, insbesondere ABS (Acrylmethyl-Butadien-Styrol-Copolymer), PC (Polycarbonat)/ABS oder PA (Polyamid).

Die Metallbeschichtung umfasst günstigerweise eine Chromschicht und/oder eine Nickelschicht und eine Kupferschicht, wobei vorteilhafterweise eine Kombination unterschiedlicher Schichten, insbesondere eine Kombination einer Chromschicht, einer Nickelschicht und einer Kupferschicht vorgesehen ist.

Die Schichtdicke der Chromschicht liegt mit Vorteil zwischen 0,5 und 1,5 µm, diejenige der Nickelschicht zwischen 2 und 20 µm und diejenige der Kupferschicht zwischen 5 und 30 µm.

Insbesondere zur Herstellung dickerer Metallschichten kann es vorteilhaft sein, eine Folie oder eine Folge von Folien mittels eines Thermo-TransferVerfahrens aufzubringen. Auf diese Weise können zum Beispiel 100 µm dicke Kupferschichten bzw. Schichtfolgen, wie Kupfer, Nickel, Chrom aufgebracht werden.

Ein günstiges derartiges Verfahren ist das sogenannte Heißprägeverfahren, bei welchem spezielle metallisierte Folien verwendet werden.

Eine Alternative besteht in der sogenannten "In-Mould-Decoration". Bei diesem Verfahren werden Folien in die Spritzgießform für den Kunststoffrahmen eingebracht. Beim anschließenden Spritzgießvorgang verbinden sich die Folien mit der entstehenden Oberfläche des Kunststoffteils und werden dann zusammen mit diesem aus der Form entnommen.

### Ausführungsbeispiel:

- Ausgegangen wird von einer Arbeitsschutzbrille mit einem umlaufenden, dem Gesicht des Trägers anliegenden Kunststoffrahmen, wie sie beispielsweise aus G 83 02 644.4 bekannt ist.
- Der Rahmen besteht aus ABS.
- Vor der Beschichtung durch Galvanisieren wird der Kunststoffrahmen mit Chromschwefelsäure vorbehandelt, um die Oberfläche aufzurauen.
- Es folgt dann eine Behandlung mit Palladiumoxolat-Lösung, um die Kunststoffoberfläche leitfähig zu machen.
- Galvanisch wird zunächst eine Kupferschicht mit einer Schichtdicke von 1µm in einem Kupfersalzbad aufgebracht.
- Anschließend wird galvanisch eine Nickelschicht mit einer Schichtdicke von 10 µm in einem Nickelsalzbad aufgebracht.
- Es folgt die Aufbringung einer Chromschicht mit einer Schichtdicke von 15 µm in einem Chromsalzbad.

Eine derartige Laserschutzbrille erreicht bei Laserbeschuss L 6 mit Dauerstrichlaser 1064 nm (NdYAG) bzw. L 7 (Impulsbetrieb). Mit dem CO₂₋Laser (10600 nm) wird DL 4 erreicht.

Herkömmliche Kunststoffe, wie PA, CP, PP, gefüllt mit Mineralien oder Metallpulvern, werden bei dieser Belastung bereits beschädigt.

## Patentansprüche

1. Laserschutzbrille bzw. Aufsetzrahmen für eine Laserschutzbrille umfassend einen Rahmen aus Kunststoff mit einer Metallbeschichtung, **dadurch gekennzeichnet, dass** die Metallbeschichtung durch Galvanisieren aufgebracht ist.

2. Laserschutzbrille nach Anspruch 1, **dadurch gekennzeichnet, dass** der Kunststoff insbesondere ABS (Acrylmethyl-Butadien-StyrolCopolymer), PC (Polycarbonat)/ABS oder PA (Polyamid) ist.

3. Laserschutzbrille nach Anspruch 1, **dadurch gekennzeichnet, dass** die Metallbeschichtung eine Chromschicht umfasst.

4. Laserschutzbrille nach Anspruch 1, **dadurch gekennzeichnet, dass** die Metallbeschichtung eine Nickelschicht umfasst.

5. Laserschutzbrille nach Anspruch 1, **dadurch gekennzeichnet, dass** die Metallbeschichtung eine Kupferschicht umfasst.

6. Laserschutzbrille nach Anspruch 1, **dadurch gekennzeichnet, dass** die Metallbeschichtung eine Mehrzahl von Schichten aus unterschiedlichen Metallen umfasst.

7. Laserschutzbrille nach Anspruch 6, **dadurch gekennzeichnet, dass** die Metallbeschichtung wenigstens eine Chromschicht, eine Nickelschicht und eine Kupferschicht umfasst.

8. Laserschutzbrille nach Anspruch 7, **dadurch gekennzeichnet, dass** die Dicke der Chromschicht 0,5 bis 1,5 µm beträgt.

9. Laserschutzbrille nach Anspruch 7, **dadurch gekennzeichnet, dass** die Dicke der Nickelschicht 2 bis 20 µm beträgt.

10. Laserschutzbrille nach Anspruch 7, **dadurch gekennzeichnet, dass** die Dicke der Kupferschicht 5 bis 30 µm beträgt.

11. Laserschutzbrille bzw. Aufsetzrahmen für eine Laserschutzbrille umfassend einen Rahmen aus Kunststoff mit einer Metallbeschichtung, **dadurch gekennzeichnet, dass** die Metallbeschichtung in Form einer Metallfolie durch ein Thermo-Transfer-Verfahren aufgebracht ist.

12. Laserschutzbrille nach Anspruch 11, **dadurch gekennzeichnet, dass** die Folie mittels eines Heißprägeverfahrens aufgebracht ist.

13. Laserschutzbrille nach Anspruch 11, **dadurch gekennzeichnet, dass** die Metallfolie in die Gießform für den Rahmen eingelegt wird.
